Europäisches Patentamt

European Patent Office

Office européen des brevets

(11) Publication number: **0 407 008 A2**

## EUROPEAN PATENT APPLICATION

(21) Application number: **90304574.8**

(22) Date of filing: **26.04.90**

(51) Int. Cl.⁵: **A61K 37/02, C07K 13/00, C12N 15/12, C12P 21/02**

The title of the invention has been amended (Guidelines for Examination in the EPO, A-III, 7.3).

(30) Priority: **29.04.89 GB 8909919**

(43) Date of publication of application:
**09.01.91 Bulletin 91/02**

(84) Designated Contracting States:
**ES GR**

(71) Applicant: **Delta Biotechnology Limited Castle Court, Castle Boulevard Nottingham NG7 1FD(GB)**

(72) Inventor: **Geisow, Michael John 115 Main Street East Bridgford, Nottinghamshire NG13 8NH(GB)**

(74) Representative: **Bassett, Richard Simon et al ERIC POTTER & CLARKSON St. Mary's Court St. Mary's Gateate Nottingham NG1 1LE(GB)**

(54) **Fibronectin fragment.**

(57) The 140 kD fragment or a variant thereof generated by the action of the enzyme cathepsin D on human plasma fibronectin is used therapeutically for wound healing, especially on the cornea.

EP 0 407 008 A2

## SMALL POLYPEPTIDES

BACKGROUND OF THE INVENTION

The invention relates to small polypeptides derived from or related to human plasma fibronectin.

The value of human plasma fibronectin in wound healing has been established by means of animal models and clinical trials in human volunteers. The efficacy of fibronectin appears to be particularly good in the treatment of corneal injuries such as arise from elective surgery, treatment for cataracts, abrasions, viral infections and alkali burns (1,2). Much of the protein for human clinical trials has been derived from the patients' own blood. This has been for reasons of concern over potential transmission of viruses such as hepatitis non A, non B and HIV, but equally because the half-life of fibronectin formulated as eye-drops is short compared with the duration of treatment. The loss of activity of the plasma protein appears to relate to adsorption to surfaces, polymerization and proteolysis by minor contaminating proteolytic activity.

A product with the efficacy of fibronectin for wound healing, especially corneal wounds, but with improved availability, stability and freedom from viral contamination would be welcomed by clinicians as an important adjunct to current wound-healing therapies. Many companies have produced wound-healing proteins or growth factor molecules which, although of very great potency in their action on cells important for healing wounds, have proved to have exceedingly short half-lives in vivo and are consequently very difficult to deliver in effective doses. Most of the manufacturers of these growth factors recognise the need for an extracellular matrix component, especially fibronectin or a fibronectin-like molecule, as a replacement for or as an adjunct to current wound healing regimens involving the application of growth factors.

Fibronectin has long been known to comprise regions of amino acid sequence or domains which perform a specialized function. Many reviews describe the approximate location of these domains in relation to the complete amino acid sequence (e.g. 3). Most of these regions have been assigned by assaying fragments of plasma fibronectin resulting from the fortuitous cleavage of the native protein by proteases. More refined analyses of the sequences involved in a given function have been carried out by means of recombinant DNA technology. So for example, the collagen-binding region of the molecule has been closely defined (4 & 4a).

Using the approach of proteolytic fragmentation combined with peptide synthesis, the major cell-binding region of fibronectin has been located first within a 120kDa fragment, then within an 11kDa fragment and finally to a 'minimal' cell-binding peptide: arginyl-glycyl-aspartic acid or 'RGD' in single letter amino acid code (3). Although this tripeptide is able to bind to cells, its affinity for its complementary cell-surface receptors is two orders of magnitude below that of the intact protein. Other regions of sequence are involved either in independent binding to cells or in modulating the affinity of the RGD sequence. In an attempt to define better the cell-binding region of fibronectin, recombinant DNA was used to locate amino acid sequences amino-terminal to the RGD sequence which improved the affinity of the resulting molecules for cells (5). However, this work established boundaries for such sequences in relation only to cell spreading activity on coated plastic tissue-culture dishes rather than activities appropriate to a wound healing molecule. Such activities would be promotion of cell migration, chemotaxis and the support of cell division.

Another difficulty was that the cell-binding regions of fibronectin in this study were produced not as free molecules but as fusion proteins with part of the bacterial enzyme $\beta$-galactosidase. These fusions are not relevant to wound healing but may have masked or, alternatively, augmented cell spreading activity to give a false impression of the importance or otherwise of the activity of the regions of fibronectin present in each fusion protein.

The inventors noticed that whereas many investigations had produced fragments of fibronectin by the application of proteases, some of which had activity in cell spreading, not all such fragments had been located within the fibronectin molecule, permitting no conclusion in detail about the importance or otherwise of cell-binding sequences. Moreover, few of these fragments had been tested beyond cell spreading to encompass the full range of activities believed to be important in wound-healing.

Thus, for example, the fragmentation of human plasma fibronectin by the lysosomal protease cathepsin D has been described (6-12) but there was no disclosure of the molecular nature of the fragments, nor of their efficacy or otherwise in the activities, previously referred to, of importance for wound healing. We have now characterised a particular cathepsin D fragment of human plasma fibronectin and found at least one therapeutic use for it.

Accordingly, one aspect of the invention provides a pharmaceutical formulation comprising the 140kDa cathepsin D fragment of fibronectin or a variant thereof.

A second aspect provides the 140kDa cathepsin D fragment of fibronectin or a variant thereof for use in therapy.

A third aspect provides the preparation of the 140kDa cathepsin D fragment of fibronectin or a variant thereof by the cultivation of an organism transformed with a nucleotide sequence coding therefor.

A fourth aspect provides a method of treating a human or other animal with the 140kDa cathepsin D fragment of fibronectin or a variant thereof.

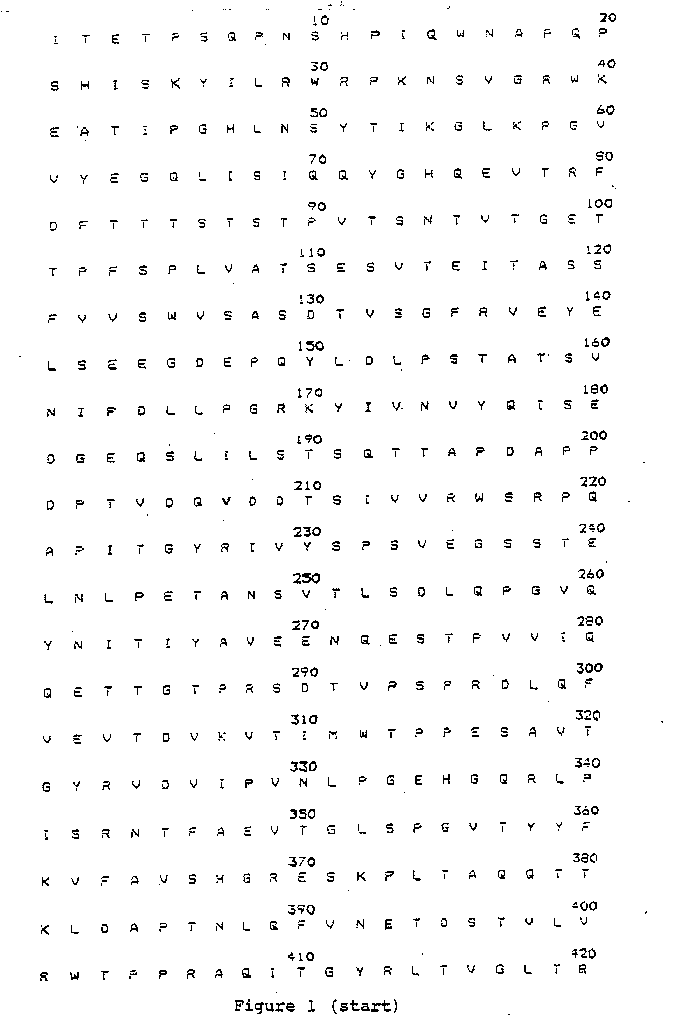

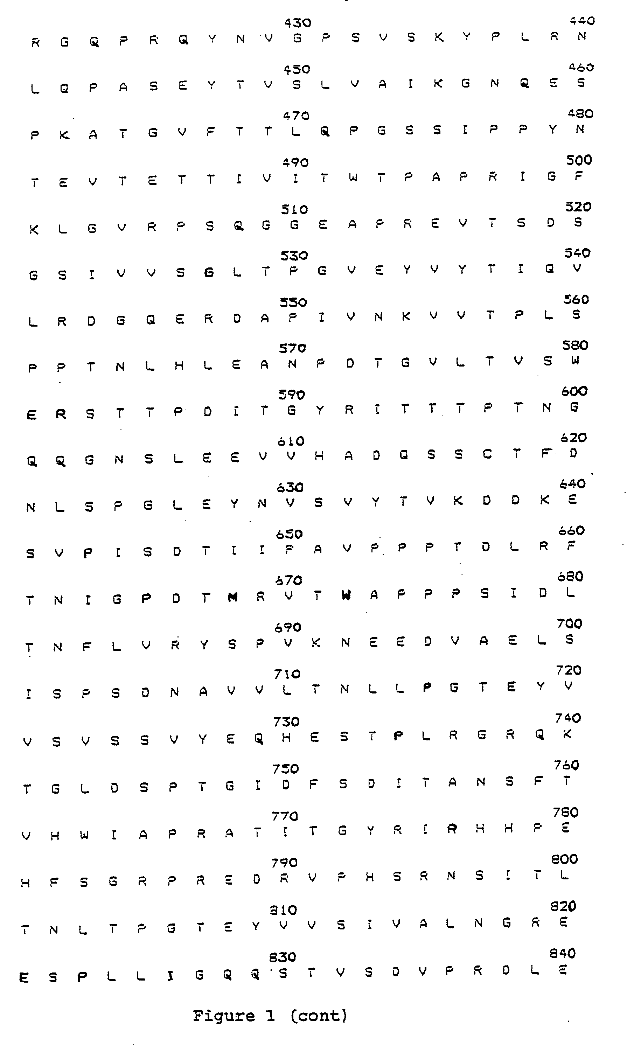

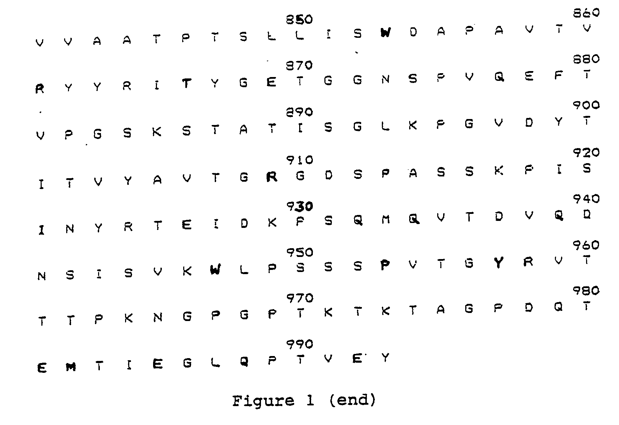

The 140kDa cathepsin fragment prepared by the process described represents the 585-1578 portion of the mature molecule, illustrated in Figure 1. However, the invention also encompasses minor variations of the sequence, such as corresponding regions of naturallyoccurring mutants (polymorphisms), variations with conservative modifications of primary sequence, slightly shorter and slightly longer molecules. In general, the compounds of the invention should comprise substantially type III homology domains Nos. 2 to 10, i.e. amino acids 669 to 1499, optionally with some or all of domains 1 and 11 (see EP-A-207 751 for a diagram of the domains of Fn).

The compounds of the invention may be formulated in known ways for delivery to the wound. For example, in the context of corneal injuries, the compounds may be dispersed in phosphate-buffered saline (preferably with a visco-elastic agent such as hyaluronic acid), a corneal bandage to go under a contact lens or a collagenous bandage.

The compounds of the invention may be made by known recombinant DNA techniques, especially by expression in transfected mammalian cells.

The compounds of the invention may be used as the sole pharmacologically active ingredient of the formulation or together with growth promoting factors or other extracellular matrix or cell adhesion factors, such as collagen, laminin, proteoglycans, tenascin, fibrin, PDGF, EGF, alpha-TGF, $\beta$-TGF and FGF (acidic or basic).

Preferred aspects of the invention will now be described by way of example and with reference to the accompanying drawings, in which:

Figure 1 (on 3 sheets) shows the amino acid sequence of Fn 585-1578, with residue 585 given as No.1;

Figures 2A to 2C show respective ELISAs of plastic-adsorbed plasma fibronectin (-[]-) and 140kDa fragment (-X-) using respective antibody preparations: (A) monoclonal antibody 4G11; (B) monoclonal antibody 3E3; (C) polyclonal anti-fibronectin antibody (±s.d.);

Figures 3A to 3C show the dependence of spreading of respective cell types on the density of adsorbed plasma fibronectin (-[]-) or 140kDa fragment (-X-), adsorption being measured in arbitrary units using the calibration curve in Fig. 2B: WI38, (B) SV40-WI38, (C) FL cells (±s.d.);

Figure 4 shows areas of spread SV40-WI38 cells on substrates containing increasing densities of adsorbed fibronectin (-[]-) or the 140kDa fragment (-X-) (±s.d.);

Figures 5A to 5B show the effect of antibodies and RGD-containing peptides on cell spreading; Fig. 5A showing the concentration dependence of inhibition by polyclonal antifibronectin of spreading of SV40-WI38 cells on plasma fibronectin and Figures B & C showing the effect of the polyclonal antifibronectin (cross-hatched in two directions), peptide GRGDS (cross-hatched SW-NE), or control (cross-hatched NW-SE), on spreading of three cell types on (B) plasma fibronectin or (C) the 140kDa fragment;

Figure 6 shows the eight constituent oligonucleotides of Linker 1;

Figure 7 shows diagrammatically the construction of plasmid pDBCF4;

Figure 8 shows the ten constituent oligonucleotides of Linker 3; and

Figure 9 shows diagrammatically the construction of plasmid pDBCF6

## EXAMPLE 1: PREPARATION OF HUMAN PLASMA FIBRONECTIN (pFn)

Human pfn is purified from donor or pooled blood plasma by freezing the plasma overnight at -20°C, followed by slow thawing at +4°C. The cryoprecipitate so formed is centrifuged at 20,000 X gav for 20 min at 4°C. The precipitate is superficially washed with ice-cold PBS and re-dissolved in 0.1M Tris HC1 pH7.5. Fibronectin is recovered by affinity chromatography on gelatin-sepharose equilibrated with the same buffer. After non-absorbed protein is completely removed by washing, fibronectin is eluted at pH5.5 at room temperature. After elution the pH is readjusted to pH7.5.

## ISOLATION OF CELL-BINDING CATHEPSIN D FRAGMENT OF PLASMA FIBRONECTIN

200mg (dry weight) of human plasma fibronectin and stabilizers were dissolved in 10ml deionized water and dialysed against 400 ml of 25mM sodium citrate pH3.5. The final concentration of protein was 4.7mg/ml

3

(E 1% = 12.6). 250μl of 40mM PMSF was added in DMSO, followed by 30μl of 1mg/ml cathepsin D in 1mM HCl. Digestion was carried out for 2 hours at 30°C. This was terminated by addition of 300μg pepstatin A in 200μl ethanol. 2ml of Tris HCl pH7.6 was added and the solution stirred until clear.

The digest was passed immediately through a 5ml gelatinagarose column (Sigma Chemical Company) equilibrated with 50mM Tris HC1 pH7.6. The flow-through fraction was collected and applied to a Mono Q (Pharmacia Ltd.) anion exchange column pre-equilibrated with the same buffer. Two major protein components were eluted with a linear salt gradient of 0 to 0.5M NaC1. The second component contained the cell-binding fragment. After dilution (four-fold) with 50mM Tris pH7.6, the fragment was rechromatographed in the same manner on Mono Q.

STORAGE

Samples were dialysed against 0.1M sucrose, 0.12M NaC1 and 10mM sodium phosphate pH7.4. The dialysed protein was lyophilised and stored at -40°C. Protein was reconstituted with deionized water to the original concentration.

EXAMPLE 2: CHARACTERISATION OF 140K CATHEPSIN D CELL-BINDING FRAGMENT OF PLASMA FIBRONECTIN

Physico-Chemical Properties

On electrophoresis under denaturing (0.1% SDS) but nonreducing conditions, a single component of relative molecular weight 144kDaltons was found. Under reducing conditions, two components of relative molecular weights 130kDaltons and 116kDaltons were formed.

Amino-Terminal Analysis

Pure, desalted fragment from two separate preparations were each subjected to amino-terminal sequence analysis using an Applied Biosystems 477A gas phase sequencer. On both occasions the unique sequence Ile-Thr-Glu-Thr-Pro-Ser-Glu-Pro-Asn-Ser was obtained. Referring to the sequence of human pfn listed in the Brookhaven Laboratory data base, this represents a fragment commencing at residue 585, produced by cleavage at a Phe-Ile bond, which is consistent with the specificity of cathepsin D. No other minor sequence was observed.

Carboxy-Terminal Sequence Analysis

The 14°K cathepsin D cell-binding fragment (1nmol) was dissolved in 50μl of sodium acetate (0.2M, pH5.5). 0.5μg carboxypeptidase Y were added and 10ul aliquots removed at 0, 2, 10, 32 and 60 min and the enzyme reaction quenched by addition to 10% triethylamine. After drying in vacuo , each timed reaction mixture was re-dissolved in water and free amino acids analysed by derivatization to form the PTC-amino acids and quantitation by h.p.l.c. (Applied Biosystems 420A/130A PTC-analyser). The C-terminus of the 740kD fragment was defined by sequence determination of a cyanogen bromide peptide. The sequence Thr-Ile-Glu-Gly-Leu-Glu-Pro-Thr-Val-Glu-Tyr demonstrated that the 140kD fragment terminated at tyrosine 1578.

Amino-Terminal Sequence of Remaining Fragments not Bound to Gelatin-Agarose

Two fragments of 71kDaltons and 61kDaltons apparent molecular weight under reducing conditions in addition to the 140kDalton cell-binding fragment were not retained by gelatin-agarose. The 71kDalton and 61kDalton fragments were sequenced as a mixture exactly as described for the 140kDalton cell-binding fragment. The unique sequence Ala-Gln-Asn-Pro-Ser-Gly-Glu was obtained, which represents the sequence of human fibronectin from residue 1583. This represents a further site of cathepsin D cleavage between

4

amino acids 1582 and 1583.

Interpretation of the Cathepsin D Fragmentation

Under the conditions described, cathepsin D appears to cleave fibronectin at residue 585, generating an amino-terminal fragment containing the collagen-binding domain, which is retained on passing the digest through gelatinagarose. The second site of cleavage at residue 1583 produces two fragments of unequal mass under reducing conditions which both have the same amino terminal sequence.

From the known properties of fibronectin and its domains, it can be predicted that the 140kDalton fragment will have no high-affinity heparin-binding sites, but may possess a low affinity binding site. High affinity binding is determined by the relative salt concentration required for elution of a fragment from heparin-agarose. Table 1 shows the effective salt concentration required for elution of fibronectin fragments from such a column.

TABLE 1

| SALT CONCENTRATION REQUIRED TO ELUTE FIBRONECTIN FRAGMENTS FROM HEPARIN-AGAROSE | | |
|---|---|---|
| Fragment | Salt Concentration (mm) | Heparin-Binding Affinity |
| 1-259 | 130 | low |
| 585-1578 | 140 | low |
| 1583-COOH-terminus | 375 | strong |

EXAMPLE 3: BINDING OF 140kDa TO TISSUE CULTURE PLASTIC

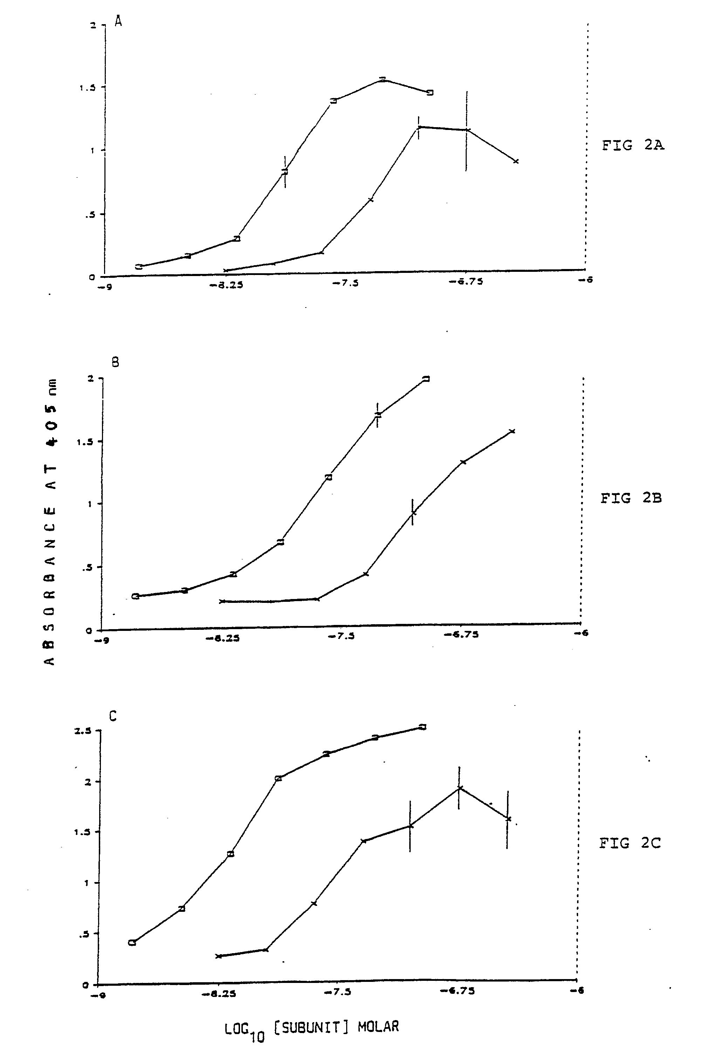

In order to assess the abilities of 140kDa and plasma fibronectin to promote cell adhesion to substrata, it was necessary to compare their affinities for tissue culture plastic. This was achieved using ELISA. Monoclonal antibody 3E3 has been shown to bind to an epitope close to the RGD site (Pierschbacher *et al.*, 1981; Carnemolla *et al.*, 1989). The site of the epitope recognised by monoclonal antibody 4G11 has not been defined. Both antibodies bound to 140kDa and to plasma fibronectin in ELISAs (Fig. 2A, B), with antibody 4G11 showing the highest affinity.

In each case the fibronectin curve is shifted to the left with respect to the 140kDa fragment (Figs. 2A, B). This could indicate a change in polypeptide conformation of the latter, arising after proteolysis and leading to lower antibody affinity. However, the shift is seen with each of the two monoclonal antibodies and also in an assay conducted with a polyclonal anti-fibronectin antibody (Fig. 2C). Rather, the data probably indicate that adsorption of the fragment is less efficient than that of intact fibronectin; a 2-4 times higher molar concentration of 140kDa is therefore required in the supernatant solution to achieve an equivalent epitope surface density.

ELISA with the polyclonal anti-fibronectin (Fig. 2C) was the most sensitive, detecting fibronectin adsorbed from solutions containing as little as 1.5μg/ml, at least two times lower than with the monoclonal antibodies. Above 5μg/ml antibody binding saturated. This occurred in a range in which the binding of the two monoclonal antibodies continued to increase, suggesting that steric inhibition of antibody binding may influence the polyclonal assay above 5μg/ml fibronectin. The same may occur in the assays using monoclonal antibody 4G11 where, at higher fibronectin concentrations, some decrease in bound antibody was observed (Fig. 2A). However, the assays were useful in indicating relative subunit densities in the range in which cellular adhesive responses occur.

EXAMPLE 4: CELL SPREADING ON THE 140kDa FRAGMENT

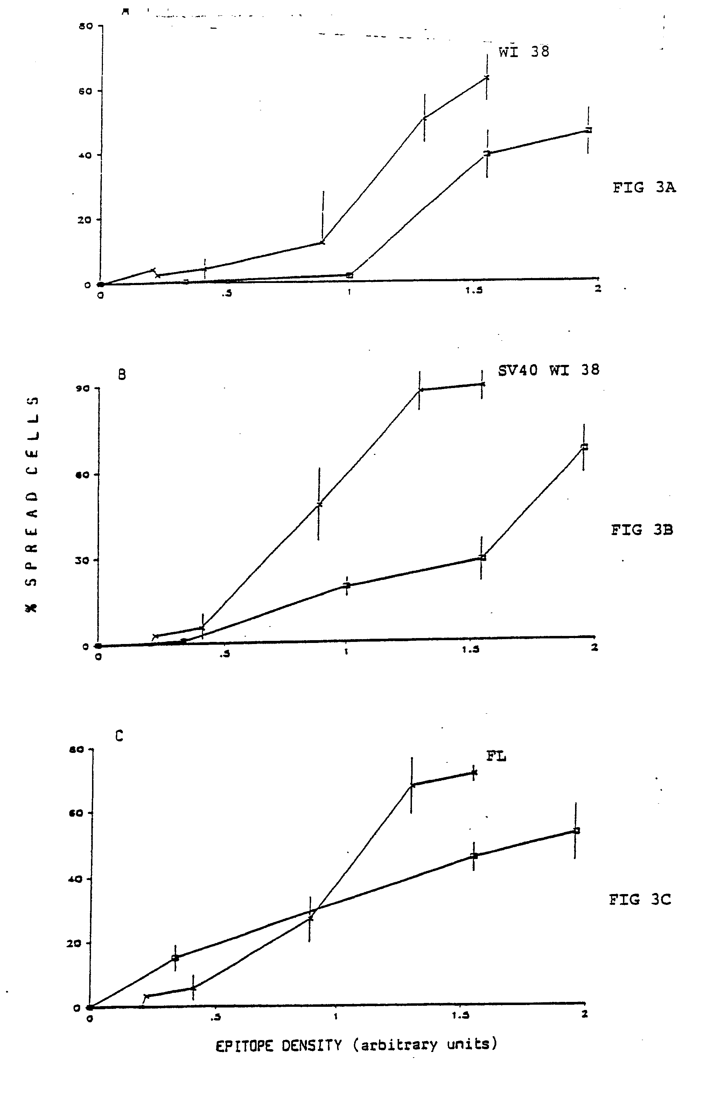

A range of cell types tested showed efficient attachment and spreading on the 140kDa fragment as well as plasma fibronectin. These included mouse L cells, bovine aortic endothelial cells, human skin fibroblast strains, human choriocarcinoma cells (BeWo), human FL amnion cells, human embryonic lung (WI38) fibroblasts and their SV40-transformed counterpart (SV40-WI38). The last three of these were selected for more detailed examination. The proportion of normal fibroblasts achieving a spread state on fibronectin or the fragment is consistently lower than in the transformed cell assays (Fig. 3A, B). The response is fibronectin density-dependent for all the cells tested (Fig. 3).

Cell spreading data were expressed in terms of relative epitope densities of adsorbed fibronectin or 140kDa obtained by immunoassay using monoclonal antibody 3E3 (Fig. 2B), thus correcting for the higher affinity of native fibronectin for tissue culture plastic. The concentration dependence of adsorption is given in Table 2. Adsorbed 140kDa is significantly more active than native plasma fibronectin in promoting spreading of normal WI38 and SV40-transformed WI38 cells (Fig. 3A, B) and at least as active as fibronectin with respect to FL cells (Fig. 3C).

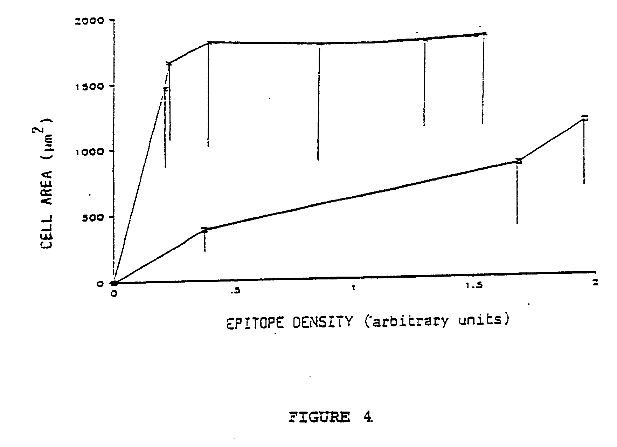

To investigate this further, area measurements were made on SV40-WI38 cells spread on fibronectin or 140kDa at various densities. In each experiment, data were selected from spread cells; cross-sectional areas of rounded (phase-bright) cells were also measured but these did not vary. The data, shown in Fig. 4, demonstrate that cells spread on 140kDa have a mean area greater than cells on fibronectin. The difference is especially marked at lower protein densities where the proportion of spread cells is low. This indicates that on low densities of 140kDa, cells tend not to spread to a reduced extent; in contrast, on intact fibronectin, net area is reduced.

No obvious differences were apparent in the morphology of cells spread on 140kDa in comparison to plasma fibronectin. Staining of actin was carried out in cells permeabilised after spreading on the two substrates. A prominent array of stress fibres was visible in each cell type within 2 hours of seeding in serum-free medium. This result shows that the signals necessary for reorganisation of actin into bundles that terminate near the inner face of the plasma membrane, previously shown to be generated in the cell response to fibronectin, are also produced rapidly in response to the truncated fragment.

EXAMPLE 5: MODULATION OF CELL RESPONSES BY ANTIBODY, PEPTIDES, HEPARIN AND DIVALENT CATIONS

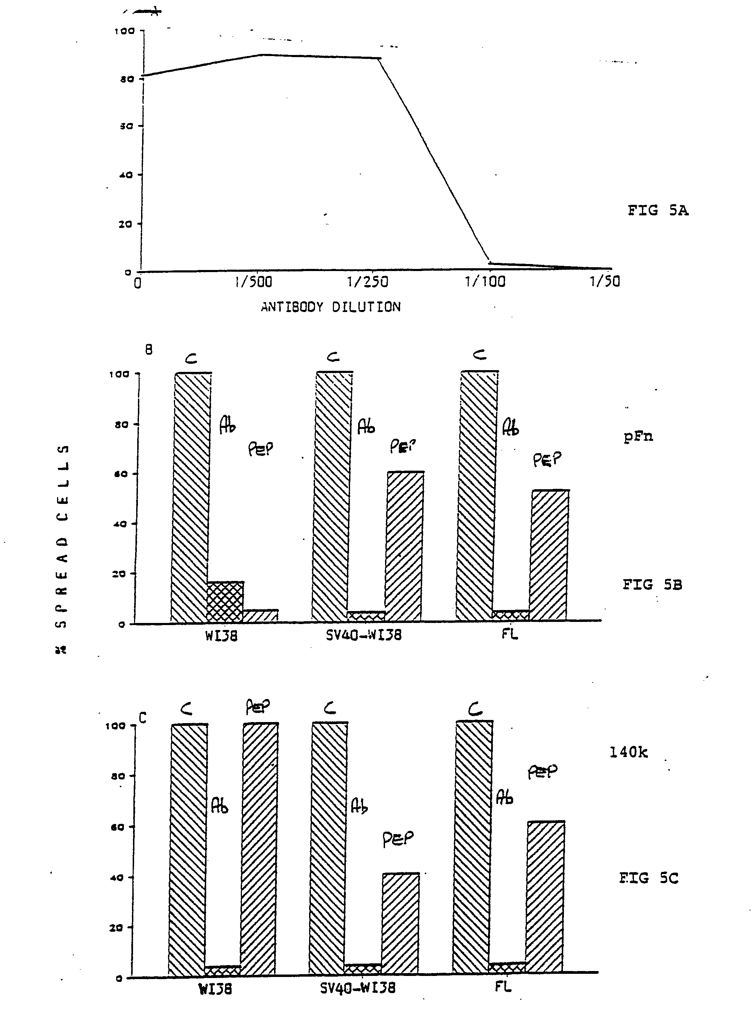

Polyclonal antibody to plasma fibronectin was able to inhibit cell adhesion on substrates containing plasma fibronectin. The active titre was 1/100 (Fig. 5A) and this was inhibitory in all three cell types tested on 140kDa (Fig. 5B). This provides an essential control for the specificity of the cell response to 140kDa.

The peptide GRGDS inhibited spreading of normal WI38 fibroblasts on fibronectin substrates. When the fibronectin was in excess (coated at $50\mu$g/ml) complete inhibition was observed at 1mg/ml peptide (Fig. 5B). Partial inhibition was observed at 0.5mg/ml (not shown). The control peptide GRGESP was inactive (not shown). Surprisingly, GRGDS added at 1mg/ml was unable to inhibit WI38 cell spreading on 140kDa even when the latter was used at much lower concentration ($25\mu$g/ml; according to Fig. 2 this should give a subunit density 4-8 fold lower than $50\mu$g/ml fibronectin). These data suggest either a conformational change at the RGD site occurring as a result of proteolysis and leading to a change in affinity for receptor, or the involvement of an independent recognition site.

GRGDS added at 1mg/ml was unable to inhibit completely spreading of SV40-WI38 cells or FL cells on plasma fibronectin, and similar results were obtained with these cell types on the 140kDa fragment.

Heparin, when added into the medium during adhesion assays, had no effect on spreading of any cell type used on fibronectin or the fragment, nor did it influence significantly the inhibitory activity of the RGD peptide.

TABLE

| Table 2. Relationship between the concentrations of plasma fibronectin or 140kDa used to coat substrata, and subsequent spreading of SV40-transformed WI38 cells. | | | | |
|---|---|---|---|---|
| The data shown are from a typical experiment. | | | | |
| | Concentration | | % spread cells | |
| | μg/ml | nM | mean | s.d. |
| Plasma fibronectin | 0 | 0 | 0 | 0 |
| | 1.0 | 4.2 | 0.8 | 2.2 |
| | 5.0 | 20.8 | 19.2 | 3.7 |
| | 10.0 | 41.7 | 28.2 | 7.9 |
| | 25.0 | 104.2 | 66.6 | 9.2 |
| | 50.0 | 208.4 | 81.5 | 6.1 |
| 140kDa | 0 | 0 | 0 | 0 |
| | 1.6 | 11.2 | 0 | 0 |
| | 3.1 | 22.3 | 3.0 | 2.6 |
| | 6.2 | 44.6 | 5.5 | 4.9 |
| | 12.5 | 89.3 | 47.9 | 13.3 |
| | 25.0 | 178.6 | 87.2 | 7.2 |
| | 50.0 | 357.1 | 89.0 | 5.3 |

## EXAMPLE 6: PRODUCTION OF FIBRONECTIN 585-1578 IN YEAST

Standard recombinant DNA procedures were as described by Maniatis et al . (1982 and 2nd edition) unless otherwise stated. Construction and analysis of M13 recombinant clones was as described by Messing (1983) and Sanger et al , (1977).

The human fibronectin coding sequence used in the construction of the following molecules is derived from the plasmid pFHDEL1 (EP-A-207 751) or by synthesis of oligonucleotides equivalent to parts of this sequence. Oligonucleotides were synthesised using phosphoramidite chemistry on an Applied Biosystems 380B oligonucleotide synthesiser according to the manufacturer's recommendations (Applied Biosystems Incorporated, Warrington, UK).

An expression vector was constructed in which DNA encoding amino acid residues 585-1578 inclusive of human fibronectin was placed downstream of the Saccharomyces cerevisiae hybrid promoter described in EP-A-258 067, and followed by a stop codon and the S.cerevisiae phosphoglycerate kinase ( PGK ) gene transcription terminator. Optionally the coding sequences may be preceded by a DNA sequence encoding a secretion signal peptide for example that of pre-pro-HSA, or yeast pre-pro alpha-factor mating signal.

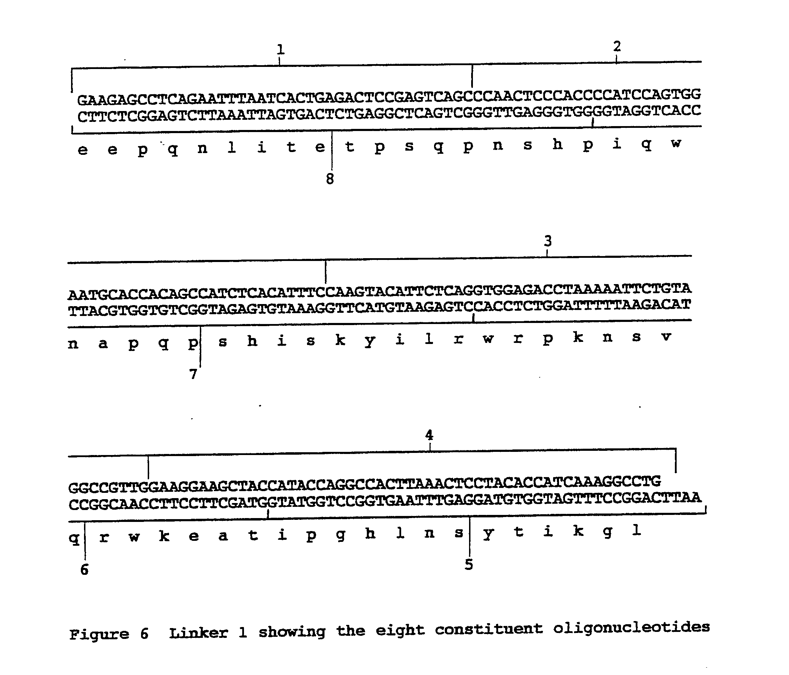

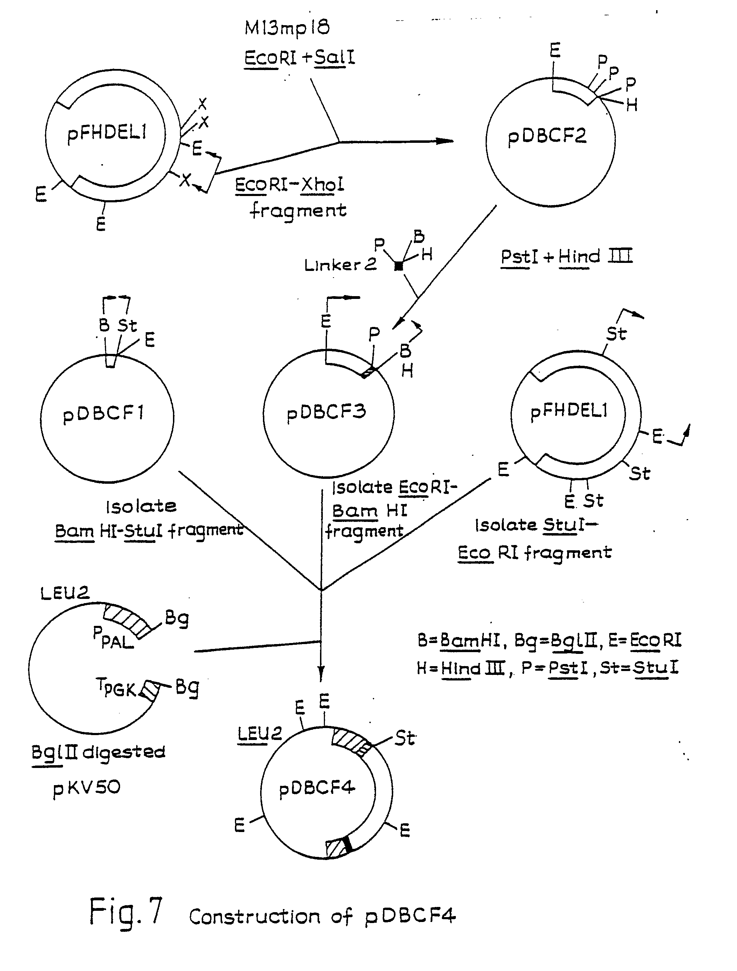

Figure 6 shows the oligonucleotides, including linker 1, used to recreate the fibronectin coding sequence from the codon for isoleucine 585 to a Stu I restriction site which includes the codon for glycine 639 of human fibronectin (Kornblihtt et al , 1985). The isoleucine codon is preceded by an ATG codon for initiation of translation, six A residues and a Bam HI cohesive end. The oligonucleotides were phosphorylated using T4 polynucleotide kinase, annealed in pairs, i.e. 1 + 8, 2 + 7, 3 + 6 and 4 + 5 and then ligated with Bam HI + Eco RI-digested M13mp19 (Norrander et al ., 1983) to form pDBCF1 (Figure 7).

The plasmid pFHDEL1 contains the complete human fibronectin coding sequence (EP-A-207 751). This plasmid was digested with Eco RI and Xho I and then a 0.77kb fragment was isolated and then ligated with Eco RI + Sall -digested M13mp18 (Norrander et al ., 1983) to form pDBCF2 (Figure 7).

The following linker, linker 2, was synthesised, representing from the Pst I site at 4790 of the fibronectin sequence (see EP-A-207 751) to the codon for valine 1578 which is followed by a stop codon (TAA), a Hin dIII site and then a Bam HI cohesive end:

Linker 2

| | G | P | D | Q | T | E | M | T | I | E | G |
|---|---|---|---|---|---|---|---|---|---|---|---|
| | GGT | CCA | GAT | CAA | ACA | GAA | ATG | ACT | ATT | GAA | GGC |
| ACGTCCA | GGT | CTA | GTT | TGT | CTT | TAC | TGA | TAA | CTT | CGA | |

| | L | Q | P | T | V | E | Y | stop | |
|---|---|---|---|---|---|---|---|---|---|
| | TTG | CAG | CCC | ACA | GTG | GAG | TAT | TAA | GCTTG |
| | AAC | GTC | GGG | TGT | CAC | CTC | ATA | ATT | CGAACCTAG |

This linker was ligated with Pst I + Hin dIII-digested pDBCF2 to form pDBCF3 (Figure 7). This plasmid was then digested with Eco RI and Bam HI and the 0.68kb Eco RI-Bam HI fragment was purified and then ligated with the 0.17kb Bam HI-Stu I fragment of PDBCF1 and the 2.22kb Stu I-Eco RI fragment of pFHDEL1 into Bgl II digested pKV50 (EP-A-258 067) to form pDBCF4 (Figure 7). This plasmid was introduced into S.cerevisiae S150-2B ( a leu2-3 leu2-112 ura3-52 trp-289 his3- 1) by standard procedures (Beggs, 1978), selecting for complementation of the leu 2 mutations by the LEU2 gene present on the plasmid. Transformants were subsequently analysed and found to produce the cathepsin D fragment of fibronectin (amino acids 585-1578) intracellularly.

Variation

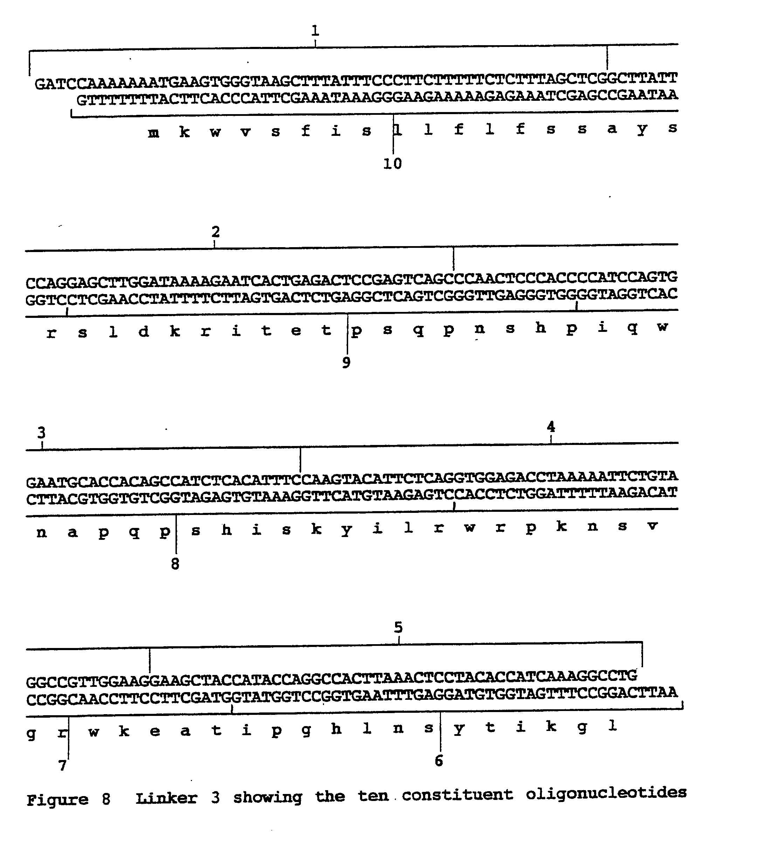

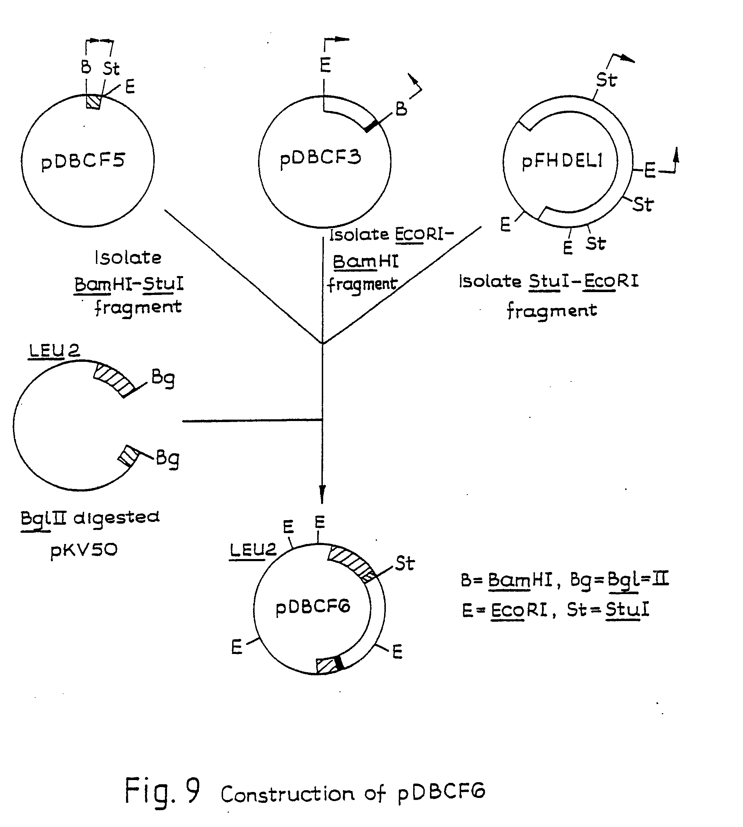

In order to direct secretion of fibronectin (585-1578) into the growth medium, a plasmid was constructed in which the fibronectin coding sequence was preceded by DNA encoding a hybrid secretion signal peptide of sequence:
M K W V S F I S L L F L F S S A Y S R S L D K R
Figure 8 shows the oligonucleotides used to create a DNA sequence encoding this secretion signal peptide in frame with DNA encoding isoleucine 585 to the Stu I site which includes the codon for glycine 639 of human fibronectin. The oligonucleotides were phosphorylated using T4 polynucleotide kinase, annealed in pairs, i.e. 1 + 10, 2 + 9, 3 + 8, 4 + 7, 5 + 6 and then ligated with Bam HI + Eco RI-digested M13mp19 (Norrander et al ., 1983) to form pDBCF5 (Figure 9). This plasmid was then digested with Bam HI and Stu I and the 0.25kb fragment was isolated and then was ligated with the 0.68kb Eco RI-Bam HI fragment of pDBCF3 and the 2.22kb Stu I-Eco RI fragment of pFHDEL1 into Bgl II-digested pKV50 to form pDBCF6 (Figure 9). This plasmid was then introduced into S.cerevisiae S150-2B wherein it directed the expression and secretion of fibronectin (585-1578).

EXAMPLE 5: TRANSFORMATION OF MAMMALIAN CELLS

The fibronectin cDNA encoding residue 585 to 1578 was available on a Bgl II restriction fragment. This can be used, following the construction of appropriate oligonucleotide linkers which maintain the coding sequence and the reading frame, in any suitable proprietary or commercial expression vector and transfected into mammalian cells, for example pMAMneo which contains the long terminal repeat enhancer of Rous sarcoma virus and steroid-sensitive promoter elements from mouse mammary tumour virus transfected into mammalian cells, for example mouse cells, whereby the expression and secretion of fibronectin 585 to 1578 can be achieved. Also within the scope of the invention is the expression of this sequence by means of transgenic animals, whereby a suitable promoter (for example the ovine β-lactoglobulin promoter of WO 88/10118) is used in conjunction with the DNA described to achieve expression of fibronectin 585 to 1578 in the milk of transgenic female animals.

REFERENCES

1. Ding, M and Burstein, N.L. (1988). J. Ocular Pharmacology , 4; 75-91.

2. EP-A-0 240 031 A1

3. Akiyama, S.K. and Yamada, K.M. (1987). Adv. Enzymol ., 57; 1-57.

4. Owens, R.J. and Baralle, F.E. (1986). EMBO J ., 5; 2825-2830.

4a. EP-A-207 751 (Delta Biotechnology Ltd).

5. Obara, M., Kang, M.S. and Yamada, K.M. (1988), Cell , 53; 649-657.

6. Postlethwaite, E.A., Keski-oja, J., Balian, G. and Kang, A.H. (1981). J. Exp. Med 153; 494-499.

7. Richter, H. and Hormann, H. (1982). Hoppe-Seyler's Z. Physiol, Chem. 363; 351-364.

8. Richter, H. and Hormann, H. (1983). FEBS Lett . 155; 317-320.

9. Markiovic, Z., Engel, J., Richter, H and Hormann, H. (1983), Hoppe-Seyler's Z. Physiol. Chem 364; 551-561.

10. Click, E.M. and Balian, G. (1985). Biochemistry , 24; 6685-6696.

11. Savill, C.M. and Ayad, S.R. (1986). Anti Cancer Research , 6; 321-326.

12. Keil-Dlouha, V. and Planchenault, T. (1986). Proc. Natl. Acad. Sci ., 83; 5377-5381.

13. Norrander et al , (1983) Gene 26 , 101-106

14. Beggs, J.D. (1978), Nature . 275; 104-109.

15. Kornblihtt, A.R., et al . (1985), EMBO J. 4; 1755-1795.

16. Maniatis, T. et al (1982), Molecular Cloning: A laboratory manual. Cold Spring Harbor Laboratory, Cold Spring Harbor, New York.

17. Messing, J. (1983), Methods Enzymol . 101; 20-78.

18. Sanger, F. et al . (1977), Proc. Natl. Acad. Sci. USA . 74; 5463-5467.

19. Pierschbacher et al . (1981), Cell 26, 259-267.

20. Carnemolla et al . (1989), J. Cell Biol. 108, 1139-1148.

**Claims**

1. A pharmaceutical formulation comprising the 140kDa cathepsin D fragment of fibronectin or a variant thereof.

2. A formulation according to Claim 1 for use on wounds.

3. A formulation according to Claim 2 for use in the eye.

4. The 140kDa cathepsin D fragment of fibronectin or a variant thereof for use in therapy.

5. A process for the preparation of the 140kDa cathepsin D fragment of fibronectin or a variant thereof by the cultivation of an organism transformed with a nucleotide sequence coding thereof.

```
                                          10                          20
   I  T  E  T  P  S  Q  P  N  S  H  P  I  Q  W  N  A  P  Q  P

                                          30                          40
   S  H  I  S  K  Y  I  L  R  W  R  P  K  N  S  V  G  R  W  K

                                          50                          60
   E  A  T  I  P  G  H  L  N  S  Y  T  I  K  G  L  K  P  G  V

                                          70                          80
   V  Y  E  G  Q  L  I  S  I  Q  Q  Y  G  H  Q  E  V  T  R  F

                                          90                         100
   D  F  T  T  T  S  T  S  T  P  V  T  S  N  T  V  T  G  E  T

                                         110                         120
   T  P  F  S  P  L  V  A  T  S  E  S  V  T  E  I  T  A  S  S

                                         130                         140
   F  V  V  S  W  V  S  A  S  D  T  V  S  G  F  R  V  E  Y  E

                                         150                         160
   L  S  E  E  G  D  E  P  Q  Y  L  D  L  P  S  T  A  T  S  V

                                         170                         180
   N  I  P  D  L  L  P  G  R  K  Y  I  V  N  V  Y  Q  I  S  E

                                         190                         200
   D  G  E  Q  S  L  I  L  S  T  S  Q  T  T  A  P  D  A  P  P

                                         210                         220
   D  P  T  V  D  Q  V  D  D  T  S  I  V  V  R  W  S  R  P  Q

                                         230                         240
   A  P  I  T  G  Y  R  I  V  Y  S  P  S  V  E  G  S  S  T  E

                                         250                         260
   L  N  L  P  E  T  A  N  S  V  T  L  S  D  L  Q  P  G  V  Q

                                         270                         280
   Y  N  I  T  I  Y  A  V  E  E  N  Q  E  S  T  P  V  V  I  Q

                                         290                         300
   Q  E  T  T  G  T  P  R  S  D  T  V  P  S  P  R  D  L  Q  F

                                         310                         320
   V  E  V  T  D  V  K  V  T  I  M  W  T  P  P  E  S  A  V  T

                                         330                         340
   G  Y  R  V  D  V  I  P  V  N  L  P  G  E  H  G  Q  R  L  P

                                         350                         360
   I  S  R  N  T  F  A  E  V  T  G  L  S  P  G  V  T  Y  Y  F

                                         370                         380
   K  V  F  A  V  S  H  G  R  E  S  K  P  L  T  A  Q  Q  T  T

                                         390                         400
   K  L  D  A  P  T  N  L  Q  F  V  N  E  T  D  S  T  V  L  V

                                         410                         420
   R  W  T  P  P  R  A  Q  I  T  G  Y  R  L  T  V  G  L  T  R
```

Figure 1 (start)

```
                              430                             440
  R   G   Q   P   R   Q   Y   N   V   G   P   S   V   S   K   Y   P   L   R   N

                              450                             460
  L   Q   P   A   S   E   Y   T   V   S   L   V   A   I   K   G   N   Q   E   S

                              470                             480
  P   K   A   T   G   V   F   T   T   L   Q   P   G   S   S   I   P   P   Y   N

                              490                             500
  T   E   V   T   E   T   T   I   V   I   T   W   T   P   A   P   R   I   G   F

                              510                             520
  K   L   G   V   R   P   S   Q   G   G   E   A   P   R   E   V   T   S   D   S

                              530                             540
  G   S   I   V   V   S   G   L   T   P   G   V   E   Y   V   Y   T   I   Q   V

                              550                             560
  L   R   D   G   Q   E   R   D   A   P   I   V   N   K   V   V   T   P   L   S

                              570                             580
  P   P   T   N   L   H   L   E   A   N   P   D   T   G   V   L   T   V   S   W

                              590                             600
  E   R   S   T   T   P   D   I   T   G   Y   R   I   T   T   T   P   T   N   G

                              610                             620
  Q   Q   G   N   S   L   E   E   V   V   H   A   D   Q   S   S   C   T   F   D

                              630                             640
  N   L   S   P   G   L   E   Y   N   V   S   V   Y   T   V   K   D   D   K   E

                              650                             660
  S   V   P   I   S   D   T   I   I   P   A   V   P   P   P   T   D   L   R   F

                              670                             680
  T   N   I   G   P   D   T   M   R   V   T   W   A   P   P   P   S   I   D   L

                              690                             700
  T   N   F   L   V   R   Y   S   P   V   K   N   E   E   D   V   A   E   L   S

                              710                             720
  I   S   P   S   D   N   A   V   V   L   T   N   L   L   P   G   T   E   Y   V

                              730                             740
  V   S   V   S   S   V   Y   E   Q   H   E   S   T   P   L   R   G   R   Q   K

                              750                             760
  T   G   L   D   S   P   T   G   I   D   F   S   D   I   T   A   N   S   F   T

                              770                             780
  V   H   W   I   A   P   R   A   T   I   T   G   Y   R   I   R   H   H   P   E

                              790                             800
  H   F   S   G   R   P   R   E   D   R   V   P   H   S   R   N   S   I   T   L

                              810                             820
  T   N   L   T   P   G   T   E   Y   V   V   S   I   V   A   L   N   G   R   E

                              830                             840
  E   S   P   L   L   I   G   Q   Q   S   T   V   S   D   V   P   R   D   L   E
```

Figure 1 (cont)

```
                                        850                          860
V   V   A   A   T   P   T   S   L   L   I   S   W   D   A   P   A   V   T   V

                                        870                          880
R   Y   Y   R   I   T   Y   G   E   T   G   G   N   S   P   V   Q   E   F   T

                                        890                          900
V   P   G   S   K   S   T   A   T   I   S   G   L   K   P   G   V   D   Y   T

                                        910                          920
I   T   V   Y   A   V   T   G   R   G   D   S   P   A   S   S   K   P   I   S

                                        930                          940
I   N   Y   R   T   E   I   D   K   P   S   Q   M   Q   V   T   D   V   Q   D

                                        950                          960
N   S   I   S   V   K   W   L   P   S   S   S   P   V   T   G   Y   R   V   T

                                        970                          980
T   T   P   K   N   G   P   G   P   T   K   T   K   T   A   G   P   D   Q   T

                                        990
E   M   T   I   E   G   L   Q   P   T   V   E   Y
```

Figure 1 (end)

EP 0 407 008 A2

FIG 2A

FIG 2B

FIG 2C

ABSORBANCE AT 405 nm

LOG$_{10}$ [SUBUNIT] MOLAR

13

FIG 3A

FIG 3B

FIG 3C

EP 0 407 008 A2

FIGURE 4

FIG 5A

FIG 5B

pFn

140k

FIG 5C

16

1　　　　　　　　　　　　　　　　　　　　　2

```
GAAGAGCCTCAGAATTTAATCACTGAGACTCCGAGTCAGCCCAACTCCCACCCCATCCAGTGG
CTTCTCGGAGTCTTAAATTAGTGACTCTGAGGCTCAGTCGGGTTGAGGGTGGGGTAGGTCACC
```

e　e　p　q　n　l　i　t　e　t　p　s　q　p　n　s　h　p　i　q　w

8

3

```
AATGCACCACAGCCATCTCACATTTCCAAGTACATTCTCAGGTGGAGACCTAAAAATTCTGTA
TTACGTGGTGTCGGTAGAGTGTAAAGGTTCATGTAAGAGTCCACCTCTGGATTTTTAAGACAT
```

n　a　p　q　p　s　h　i　s　k　y　i　l　r　w　r　p　k　n　s　v

7

4

```
GGCCGTTGGAAGGAAGCTACCATACCAGGCCACTTAAACTCCTACACCATCAAAGGCCTG
CCGGCAACCTTCCTTCGATGGTATGGTCCGGTGAATTTGAGGATGTGGTAGTTTCCGGACTTAA
```

q　r　w　k　e　a　t　i　p　g　h　l　n　s　y　t　i　k　g　l

6　　　　　　　　　　　　　　　　　　　　5

Figure 6　Linker 1 showing the eight constituent oligonucleotides

17

Fig. 7 Construction of pDBCF4

1

GATCCAAAAAAATGAAGTGGGTAAGCTTTATTTCCCTTCTTTTTTCTCTTTAGCTCGGCTTATT
GTTTTTTTACTTCACCCATTCGAAATAAAGGGAAGAAAAAGAGAAATCGAGCCGAATAA

m   k   w   v   s   f   i   s   l   l   f   l   f   s   s   a   y   s

10

2

CCAGGAGCTTGGATAAAAGAATCACTGAGACTCCGAGTCAGCCCAACTCCCACCCCATCCAGTG
GGTCCTCGAACCTATTTTCTTAGTGACTCTGAGGCTCAGTCGGGTTGAGGGTGGGGTAGGTCAC

r   s   l   d   k   r   i   t   e   t   p   s   q   p   n   s   h   p   i   q   w

9

3                                                                4

GAATGCACCACAGCCATCTCACATTTCCAAGTACATTCTCAGGTGGAGACCTAAAAAATTCTGTA
CTTACGTGGTGTCGGTAGAGTGTAAAGGTTCATGTAAGAGTCCACCTCTGGATTTTTTAAGACAT

n   a   p   q   p   s   h   i   s   k   y   i   l   r   w   r   p   k   n   s   v

8

5

GGCCGTTGGAAGGAAGCTACCATACCAGGCCACTTAAACTCCTACACCATCAAAGGCCTG
CCGGCAACCTTCCTTCGATGGTATGGTCCGGTGAATTTGAGGATGTGGTAGTTTCCGGACTTAA

g   r   w   k   e   a   t   i   p   g   h   l   n   s   y   t   i   k   g   l

7                                                                6

Figure 8   Linker 3 showing the ten constituent oligonucleotides

Fig. 9 Construction of pDBCF6